# EUROPEAN PATENT APPLICATION

(11) **EP 2 517 694 A1**
(43) Date of publication of application: **31.10.2012**
(21) Application number: 10839384.4
(22) Date of filing: 21.12.2010
(51) Int. Cl.: A61K 8/64, A61K 8/06, A61Q 5/02, A61Q 5/12

(54) **MANUFACTURING METHOD FOR HAIR COSMETIC**

(30) Priority: 21.12.2009 JP 2009289290
(71) Applicant: FUJIFILM Corporation, Tokyo 106-0031 (JP)
(72) Inventor: KANAZAWA Katsuhiko, Ashigarakami-gun Kanagawa 258-8577 (JP); AIMI Makiko, Ashigarakami-gun Kanagawa 258-8577 (JP); TANAKA Hideaki, Ashigarakami-gun Kanagawa 258-8577 (JP)
(74) Representative: Grünecker, Kinkeldey, Stockmair & Schwanhäusser
(86) International application number: PCT/JP2010/072977
(87) International publication number: WO 2011/078154

(57) **Abstract**

It is an object of the present invention to provide a method for producing a hair cosmetic product comprising protein nanoparticles which is obtained by mixing an aqueous medium and an oil medium, wherein the method enables stable emulsification without losing the performance of the protein nanoparticles. The present invention provides a method for producing a hair cosmetic product containing protein nanoparticles which is obtained by mixing an aqueous medium and an oil medium, wherein (a) protein nanoparticles having a mean particle size of 10 to 200 nm are mixed with an aqueous medium having a pH value different from the isoelectric point of the protein at an amount of 0.01% by weight to 5% by weight based on the total weight of the entire solution; and (b) the obtained liquid is mixed with a substance selected from a cationic substance and an anionic substance, which has an electric charge that is on the same side as that of the protein obtained in the step (a) .

## Description

### Technical Field

The present invention relates to a method for producing a hair cosmetic product. More specifically, the present invention relates to a method for producing a hair cosmetic product comprising protein nanoparticles which is obtained by mixing an aqueous medium and an oil medium.

### Background Art

A hair treatment product is used mainly for the purpose of favorably changing hair texture or appearance. This hair treatment product is generally a one-agent product. In order to favorably change hair texture or appearance, a composition containing, as main ingredients, a cationic active agent, oil, and silicone has been used. However, the hair treatment product has been problematic in that such a favorable change in hair due to such a composition is obtained only temporarily, and in that it disappears as a result of hair-washing.

Patent Document 1 discloses that a particulate substance having a mode value of primary particle size of colloidal metal, such as silica, a crosslinked polymer, alumina, aluminum silicate or titanium dioxide, that is 7 to 40 nm, exhibits an effect of increasing body in hair or an effect of styling hair. However, this particulate substance is problematic in that it needs to be a colloidal particle having a Young's modulus of at least 4 Gpa, and it is problematic in terms of safety such as the metabolism of the colloidal metal.

Patent Document 2 discloses that the performance of a treatment product is improved by adding calcium carbonate having a size of 10 to 250 nm to the treatment product. However, as with Patent Document 1, this treatment product is problematic in terms of the safety of colloidal particles.

Patent Document 3 discloses that an active ingredient is deposited on protein nanoparticles dispersed in water, and that the particles can solubilize the hydrophobic active ingredient and can deliver it. However, Patent Document 3 relates to water-dispersed protein nanoparticles. Thus, when an excessive amount of fat-soluble substance was added, or when an ionic surfactant and the like were added to further improve the performance of a treatment product, electrostatic coagulation/sedimentation occurred, and a stable system could not be established. In Patent Document 3, since the nanoparticles dispersed in an aqueous medium are protein nanoparticles, they have an isoelectric point. It can be confirmed that when the pH value is less than the isoelectric point, the nanoparticles are positively charged, and that when the pH value is greater than the isoelectric point, the nanoparticles are negatively charged. Accordingly, the protein nanoparticles of Patent Document 3 are problematic in that, when an anionic compound is added at low pH, coagulation/sedimentation occurs, and when a cationic compound is added at high pH, coagulation/sedimentation occurs and the system is in an instable condition.

### Prior Art Documents

### Patent Documents

[Patent Document 1] International Publication WO01/030310
[Patent Document 2] JP Patent Publication (Kohyo) No. 2004-532256 A
[Patent Document 3] JP Patent Publication (Kokai) No. 2008-201767 A

### Summary of Invention

### Object to be Solved by the Invention

It is an object of the present invention to provide a method for producing a hair cosmetic product comprising protein nanoparticles which is obtained by mixing an aqueous medium and an oil medium, wherein the method enables stable emulsification without losing the performance of the protein nanoparticles. More specifically, it is an object of the present invention to provide a method for producing a hair cosmetic product comprising protein nanoparticles which is obtained by mixing an aqueous medium and an oil medium, wherein the method is able to suppress coagulation (separation of a system) generated when the protein nanoparticles are mixed with an ionic (cationic or anionic) substance.

### Means for Solving the Object

As a result of intensive studies directed towards achieving the aforementioned object, the present inventors have found that coagulation (separation of a system) can be suppressed and a stable emulsion can be prepared by mixing protein nanoparticles with an aqueous medium having a pH value less than the isoelectric point of the protein so that the protein nanoparticles are positively charged, and then by mixing them with a cationic substance, or in contrast to the aforementioned operations, by mixing protein nanoparticles with an aqueous medium having a pH value greater than the isoelectric point of the protein so that the protein nanoparticles are negatively charged, and then by mixing them with an anionic substance. Based on these findings, the present invention has been completed.

The present invention provides a method for producing a hair cosmetic product containing protein nanoparticles which is obtained by mixing an aqueous medium and an oil medium, wherein (a) protein nanoparticles having a mean particle size of 10 to 200 nm are mixed with an aqueous medium having a pH value different from the isoelectric point of the protein at an amount of 0.01% by weight to 5% by weight based on the total weight of the entire solution; and (b) the obtained liquid is mixed with a substance selected from a cationic substance and an anionic substance, which has an electric charge that is on the same side as that of the protein obtained in the step (a).

Preferably, in the step (a), the protein nanoparticles having a mean particle size of 10 to 200 nm are mixed with an aqueous medium having a pH value less than the isoelectric point of the protein at an amount of 0.01 % by weight to 5% by weight based on the total weight of the entire solution, and in the step (b), the obtained liquid is mixed with a cationic substance.

Preferably, in the step (a), the protein nanoparticles having a mean particle size of 10 to 200 nm are mixed with an aqueous medium having a pH value greater than the isoelectric point of the protein at an amount 0.01% by weight to 5% by weight based on the total weight of the entire solution, and in the step (b), the obtained liquid is mixed with an anionic substance.
Preferably, the hair cosmetic product is a hair wash, a treatment, a conditioner, or a rinse.

Preferably, in the step (b), the liquid obtained in the step (a) is mixed with an oil medium and a cationic or anionic substance, so as to prepare an emulsion.
Preferably, in the step (a), the protein nanoparticles having a mean particle size of 10 to 200 nm are mixed with either an aqueous medium having a pH value less than the isoelectric point of the protein by pH 0.5 or more, or an aqueous medium having a pH value greater than the isoelectric point of the protein by pH 0.5 or more, at an amount of 0.01% by weight to 5% by weight based on the total weight of the entire solution, so as to prepare an emulsion.

Preferably, in the step (a), the protein nanoparticles having a mean particle size of 10 to 200 nm are mixed with an aqueous medium having a pH value different from the isoelectric point of the protein at an amount of 0.1% by weight to 5% by weight based on the total weight of the entire solution, so as to prepare an emulsion.

Preferably, the protein is at least one type selected from the group consisting of collagen, gelatin, acid-treated gelatin, albumin, ovalbumin, casein, a casein derivative, transferrin, globulin, fibroin, fibrin, laminin, fibronectin, and vitronectin.

Preferably, the method of the present invention further comprises a step of mixing a silicon compound into the hair cosmetic product.
Preferably, the method of the present invention further comprises a step of mixing a coloring agent into the hair cosmetic product.

### Effect of the invention

According to the present inventive method for producing a hair cosmetic product comprising protein nanoparticles which is obtained by mixing an aqueous medium and an oil medium, a stable emulsion can be prepared without losing the performance of the protein nanoparticles.

### Brief Description of Drawings

[Figure 1] Figure 1 shows the results obtained by observing under an electron microscope, untreated damaged hair (human hair) and damaged hair (human hair) where casein solution 4 or casein solution 5 was applied, penetrated and removed
[Figure 2] Figure 2 shows the results obtained by observing under an optical microscope and a scanning electron microscope (SEM), untreated damaged hair (human hair) and damaged hair (human hair) where the emulsion obtained in Example 2 was applied and penetrated, followed by washing and drying and where Pt was deposited by evaporation.

### Embodiments for Carrying out the Invention

Hereinafter, the embodiments for carrying out the present invention will be described in detail.
The present invention relates to a method for producing a hair cosmetic product containing protein nanoparticles which is obtained by mixing an aqueous medium and an oil medium, wherein (a) protein nanoparticles having a mean particle size of 10 to 200 nm are mixed with an aqueous medium having a pH value different from the isoelectric point of the protein at an amount of 0.01% by weight to 5% by weight based on the total weight of the entire solution; and (b) the obtained liquid is mixed with a substance selected from a cationic substance and an anionic substance, which has an electric charge that is on the same side as that of the protein obtained in the step (a).

The present invention includes the following two embodiments.

### (First Embodiment)

In the step (a), the protein nanoparticles having a mean particle size of 10 to 200 nm are mixed with an aqueous medium having a pH value less than the isoelectric point of the protein at an amount of 0.01% by weight to 5% by weight based on the total weight of the entire solution so as to prepare an emulsion, and in the step (b), the obtained liquid is mixed with a cationic substance.

### (Second Embodiment)

In the step (a), the protein nanoparticles having a mean particle size of 10 to 200 nm are mixed with an aqueous medium having a pH value greater than the isoelectric point of the protein at an amount of 0.01% by weight to 5% by weight based on the total weight of the entire solution so as to prepare an emulsion, and in the step (b), the obtained liquid is mixed with an anionic substance.

The object of the present invention is to suppress coagulation (separation of a system) generated when the protein nanoparticles are mixed with a cationic or anionic substance. In the present invention, the protein is positively charged by adjusting the pH of a solution of the protein nanoparticles at a pH value less than the isoelectric point of the protein, thereby providing the effect of suppressing coagulation even in a case in which the protein is mixed with a cationic substance (positive). Similarly, in the present invention, the protein is negatively charged by adjusting the pH of a solution of the protein nanoparticles at a pH value greater than the isoelectric point of the protein, thereby providing the effect of suppressing coagulation even in a case in which the protein is mixed with an anionic substance (negative).

As an example of the present invention, an emulsion is prepared by mixing an aqueous medium and an oil medium. However, the timing of preparing an emulsion is not particularly limited. The hair cosmetic product of the present invention can be produced by the following procedures, for example.

### (Examples 1)

Protein nanoparticles are mixed with an aqueous medium having a pH value different from the isoelectric point of the protein, and the obtained mixture used as a water phase is then mixed with an oil phase to prepare an emulsion. Thereafter, a substance selected from a cationic substance and an anionic substance is added to the emulsion.

### (Example 2)

Protein nanoparticles are mixed with an aqueous medium having a pH value different from the isoelectric point of the protein, and the obtained mixture used as a water phase is then mixed with an oil phase (containing a substance selected from a cationic substance and an anionic substance) to prepare an emulsion.

### (Example 3)

A water phase is mixed with an oil phase to prepare an emulsion. A cationic substance is added to the emulsion. Thereafter, protein nanoparticles that have been mixed with an aqueous medium having a pH value different from the isoelectric point of the protein are further added to the emulsion.

### (Example 4)

A water phase is mixed with an oil phase (containing a substance selected from a cationic substance and an anionic substance) to prepare an emulsion. Thereafter, protein nanoparticles that have been mixed with an aqueous medium having a pH value different from the isoelectric point of the protein are further added to the emulsion.

Preferably, in the step (a) of the present invention, protein nanoparticles having a mean particle size of 10 to 200 nm are mixed with any one of an aqueous medium having a pH value less than the isoelectric point of the protein by pH 0.5 or more and an aqueous medium having a pH value greater than the isoelectric point of the protein by pH 0.5 or more. For instance, when the used protein is casein, since the isoelectric point of casein is generally approximately pH 4.6, the casein nanoparticles are preferably mixed with an aqueous medium having a pH value less than pH 4.1, or with an aqueous medium having a pH value greater than pH 5.1.

In the step (a), when the protein nanoparticles having a mean particle size of 10 to 200 nm are mixed with an aqueous medium having a pH value different from the isoelectric point of the protein, the protein nanoparticles are added at an amount 0.01% by weight to 5% by weight based on the total weight of the entire solution. The additive amount of the protein nanoparticles is preferably 0.1% by weight to 5% by weight, and particularly preferably 0.1% by weight to 2% by weight. In the present invention, by determining the amount of the protein nanoparticles within the aforementioned range, the hair treating effect of the protein nanoparticles can be sufficiently obtained, and at the same time, coagulation (separation of a system) generated when the protein nanoparticles are mixed with an ionic (cationic or anionic) substance can be suppressed.

The protein used in the present invention may be any one of a plant-derived protein, an animal-derived protein, an organic synthetic product, and a material that can be produced by gene recombination. Examples of such an animal, from which the present protein is derived, include mammals such as bovine, goats, buffalos, swine, horses, sheep and humans. Moreover, even if the used protein is prepared by organic synthesis or gene recombination, the type of the protein is not particularly limited, as long as it has a protein structure. Such proteins may be used either singly or in combination.

Examples of the protein used in the present invention include a protein and a salt thereof which is selected from the group consisting of collagen, gelatin, acid-treated gelatin, albumin, ovalbumin, casein, casein sodium, transferrin, globulin, fibroin, fibrin, laminin, fibronectin, and vitronectin.

The casein used in the present invention is composed of at least two types selected from among α-casein, β-casein and κ-casein. When these caseins are mixed, the mixed casein comprises: α-casein at a percentage of 30% or more, and more preferably 40% or more; β-casein at a percentage of 15% or more, and more preferably 20% or more; and κ-casein at a percentage of 3% or more, and more preferably 5% or more. The α-casein may be composed of α (S1)-casein and α (S2)-casein, or may also be either single α (S1)-casein or single α (S2)-casein.

The protein that forms nanoparticles may be either crosslinked or uncrosslinked. When the protein that forms nanoparticles is crosslinked, any one of chemical crosslinking, physical crosslinking and biological crosslinking may be adopted. When a crosslinker is used, it may preferably be added at an amount of 0.1% by mass to 100% by mass based on the total mass of the casein or the salt thereof. The amount of such a crosslinker added is not limited, as long as it allows the casein to form nanoparticles.

The mean particle size of the protein nanoparticles in the present invention is generally 10 to 200 nm, preferably 10 to 100 nm, more preferably 10 to 50 nm, and particularly preferably 20 to 50 nm.

In the present invention, protein nanoparticles having a mean particle size of 10 to 200 nm, which are prepared by the following steps (a) and (b), can be used:
(a) a step of mixing a protein or a salt thereof with a basic aqueous medium with a pH value greater than pH 7; and
(b) a step of decreasing the pH of the solution obtained in the step (a) to a pH value that is apart from the isoelectric point of the protein by pH 1 or more, while stirring the solution.

In the present invention, protein nanoparticles having a mean particle size of 10 to 200 nm, which are prepared by the following steps (a) and (b), can be used:
(a) a step of mixing a protein or a salt thereof with an acidic aqueous medium with a pH value less than the isoelectric point of the protein; and
(b) a step of adjusting the pH of the solution obtained in the step (a) to a pH value that is apart from the isoelectric point of the protein by pH 0.5 or more, while stirring the solution.

The methods of preparing a dispersed solution in the present invention include: a method, which comprises mixing a protein or a salt thereof with a basic aqueous medium solution and then adjusting the pH of the obtained solution, while stirring the solution; and a method, which comprises mixing a protein or a salt thereof with an acidic aqueous medium solution with a pH value less than the isoelectric point of the protein and then adjusting the pH of the obtained solution, while stirring the solution.

Addition dropwise of an acid is simple and preferable as a method comprising mixing casein or a salt thereof with a basic aqueous medium solution and then decreasing the pH of the obtained solution while stirring the solution. However, the method is not particularly limited, as long as it satisfies solubility, temperature, and stirring condition.

Addition dropwise of a base is simple and preferable as a method comprising mixing casein or a salt thereof with an acidic aqueous medium solution and then increasing the pH of the obtained solution while stirring the solution. However, the method is not particularly limited, as long as it satisfies solubiliy, temperature, and stirring condition.

The temperature of each aqueous medium can be set as appropriate. The temperature of the aqueous medium can be set generally from 0°C to 80°C, and preferably from 25°C to 70HC. The stirring speed can be set as appropriate. It can be set from 100 rpm to 3000 rpm, and preferably from 200 rpm to 2000 rpm.

As an aqueous medium used in the present invention, an aqueous solution of organic acid, organic base, inorganic acid or inorganic base, or a buffer can be used.

A specific example of the aqueous medium used in the present invention is an aqueous solution containing the following: organic acids such as citric acid, ascorbic acid, gluconic acid, carboxylic acid, tartaric acid, succinic acid, acetic acid, phthalic acid, trifluoroacetic acid, morpholinoethanesulfonic acid, or 2-[4-(2-hydroxyethyl)-1-piperazinyl]ethanesulfonic acid; organic bases such as tris(hydroxymethyl), aminomethane, or ammonia; inorganic acids such as hydrochloric acid, perchloric acid, or carbonic acid; and inorganic bases such as sodium phosphate, potassium phosphate, calcium hydroxide, sodium hydroxide, potassium hydroxide, or magnesium hydroxide. However, examples are not limited thereto.

The concentration of the aqueous medium used in the present invention is preferably from approximately 10 mM to approximately 1 M, and more preferably from approximately 20 mM to approximately 200 mM.

The pH of the basic aqueous medium used in the present invention is preferably pH 8 or greater, more preferably pH 8 to 12, and further preferably pH 10 to 12. If the pH is too high, it causes the possible occurrence of hydrolysis or the risk of handling. Thus, the above-described pH range is preferable.

In the present invention, the temperature at which the protein is mixed with a basic aqueous medium having pH 8 or greater is preferably 0°C to 80°C, more preferably 10°C to 70°C, and further preferably 30°C to 70°C.

The pH of the acidic aqueous medium used in the present invention is preferably pH 1.5 to 6, and more preferably pH 1.5 to 4. The particle size tends to become large at pH other than the above-described pH range.

As a means for adding a fat-soluble substance into a formulation as in the present invention, an emulsification method is preferably applied. The emulsification method is not limited, and either chemical emulsification or physical emulsification may be applied. The emulsification method applied in the present invention is preferably physical emulsification performed in a system in which shearing or pressure is given using a disperser or a homogenizer. In addition, upon emulsification, a water phase is mixed with an oil phase. Of these phases, the water phase is preferably used as a phase that has previously contained the above-described protein nanoparticles.

The oil phase is composed of an oil medium. Examples of the oil medium that can be used in the present invention include polyoxyethylene alkyl ether, fatty acid ester, fatty acid glyceryl, fatty acid polyoxyethylene glycol, fatty acid polyoxyethylene glyceryl, fatty acid polyoxyethylene hydrogenated castor oil, higher alcohol, vegetable oil, polyhydric alcohol, hydrocarbon, fluorine oil, and silicone oil. Further, taking into consideration the dispersibility of the system, a surfactant may be added to the oil phase. Specific examples of the surfactant include, but are not limited to, cetanol, stearyl alcohol, glyceryl stearate, polyethylene glycol tristearate, polyglyceride stearate, dimethicone, dimethiconol, behentrimonium chloride, behenamidopropyl dimethylamine, and a mixture thereof.

With regard to emulsification conditions, both the oil phase and the water phase are preferably emulsified and mixed with each other, while they are each in a fused state. The temperature condition is not particularly limited. It is generally 50°C to 95°C, and more preferably 60°C to 85°C. The number of rotations is not particularly limited, either, as long as it enables emulsification. It is generally 1000 to 5000 rpm, and for example, 2000 rpm to 3500 rpm. The rotation time is not particularly limited, either, as long as it enables emulsification. On a laboratory scale, the rotation time is generally 1 to 10 minutes, and preferably 3 to 5 minutes. On a production scale, it is generally 1 to 30 minutes, and preferably 3 to 20 minutes.

Protein nanoparticles are preferably prepared in a water phase, as described above. In the present invention, the pH of the water phase is changed depending on the electric charge of a substance contained in the oil phase. That is to say, in a case in which the oil phase contains a large amount of positively charged substance, the pH is adjusted to a pH value at which the protein nanoparticles contained in the water phase are positively charged. On the other hand, in a case in which the oil phase contains a large amount of negatively charged substance, the pH is adjusted to a pH value at which protein nanoparticles contained in the water phase are negatively charged.

The form of the hair cosmetic product of the present invention is not particularly limited. Examples of the form of the present hair cosmetic product include a liquid agent for external use, an aerosol, a lotion, a tonic, a liniment, an emulsion, a suspension, a saturant, a foaming agent, a skin lotion, a pack, and a sheet agent for external use.

Ingredients contained in the hair cosmetic product of the present invention are not particularly limited. For example, such ingredients can be selected from ingredients for cosmetic products and ingredients for pharmaceutical products. Examples of the ingredients for cosmetic products include an emollient, a treatment agent, a lubricant, a moisturizer, a hair restorer, a hair growth stimulant, a hair growing agent, an anti-white hair agent, an anti-aging agent, an antioxidant, a collagen synthesis promoting agent, a vitamin agent, a perfume, a coloring agent, an anhidrotic, a cooling agent, a refrigerant, and a calefacient. Examples of the ingredients for pharmaceutical products include a hair restorer, a hair growth stimulant, a hair growing agent, an antibiotic, a fungicide, an anti-inflammatory agent, an anti-allergic agent, a hormonal agent, a skin disease treating agent, an antifungal agent, an antipruritic agent, a sweater, an anhidrotic, a percutaneous absorption promoting agent, a blood circulation promoting agent, a vasoconstrictor, a vasodilator, a vitamin agent, a polypeptide, a hormone, and a skin softener. The above-mentioned active ingredients may be used singly or in combination of two or more types.

The hair cosmetic product of the present invention may comprise an ionic compound having a cationic portion used for the purpose of adsorption on hair, or a compound that utilizes hydrophobic adsorption based on the hydrophobicity of hair. Such a compound may be a polymer, a monomer, or a protein hydrolysate. As far as a compound has a cationic portion, it may be a compound having double ionicity, such as betaine. Thus, the type of the aforementioned compound is not particularly limited.

The hair cosmetic product of the present invention may preferably comprise a cationic surfactant for further improving hair texture, in addition to the above-described protein nanoparticles. As such a cationic surfactant, a mono-long-chain-alkyl quaternary ammonium salt is preferable. Specific examples of the cationic surfactant include cetyl trimethyl ammonium chloride, stearyl trimethyl ammonium chloride, alkyl trimethyl ammonium chloride, and behenyl trimethyl ammonium chloride. Of these, behenyl trimethyl ammonium chloride is particularly preferable. The cationic surfactants may be used in combination of two or more types. From the viewpoint of the further improvement of flexibility, smoothness, stability and the like, the cationic surfactant(s) are used at an amount of 0.01% by weight to 20.0% by weight, and particularly preferably 0.1% by weight to 10.0% by weight, based on the total weight of the hair cosmetic product of the present invention.

In order to further improve hair texture and the effect of preventing hair snarling, a silicone compound may also be added to the hair cosmetic product of the present invention. Examples of the silicone compound include highly polymerized dimethylpolysiloxane, dimethylpolysiloxane, amino modified silicone, and cyclic silicone. When a silicone compound is mixed into the hair cosmetic product of the present invention, it is mixed at an amount of preferably 0.1% by weight to 15% by weight, and particularly preferably 0.5% by weight to 10% by weight, based on the total weight of the present hair cosmetic product. The silicone compound may be either a polymer or a monomer. In addition, a lubricant having a cationic portion in a molecule thereof, which is used to improve the amount of the hair cosmetic product remaining in hair, may also be used, and the type of such a lubricant is not particularly limited.

The hair cosmetic product of the present invention may further comprise a coloring agent. As such a coloring agent, either a coloring agent for staining the product itself, or a coloring agent having stainability can be used. Moreover, the coloring agent having stainability may be any one of an acidic dye, a basic dye, a nitro dye (HC dye), p-phenylenediamine and a chelate compound, which can be used in cosmetic products and quasi drugs.

The hair cosmetic product of the present invention may further comprise an additive. The type of the additive is not particularly limited. One or more types selected from among a moisturizer, a softener, a pearlescent material, an antiseptic, an antioxidant, a thickener, a perfume and a pH adjuster may be used as such additive(s), Furthermore, higher alcohol (cetanol, stearyl alcohol, etc.), oils and fats from animals and/or plants, benzyl alcohol, hydroxyethyl cellulose, and the like may also be added to the hair cosmetic product, as necessary.

The hair cosmetic product of the present invention can be provided in the form of a cosmetic product or a quasi drug product, for example. Examples of the form of the hair cosmetic product of the present invention include, but are not limited to: hair styling agents such as gel, hair mousse or wax; hair washing agents such as a shampoo or a rinse-in shampoo; conditioning agents such as a rinse, a conditioner or a treatment; hair coloring agents such as a permanent hair dye, a semi-permanent hair dye or a temporary hair dye; generally so-called hair restorers such as a hair skin improver, an anti-inflammatory agent, a blood circulation promoting agent, a hair restorer, a hair growth stimulant or a depilation suppressing agent; and other products such as a hair pack, a hair liquid, a hair tonic, a hair spray, a pomade or a composition for permanent wave. The form of the hair cosmetic product of the present invention is particularly preferably a shampoo, a rinse, a conditioner, or a treatment (an in-bath type treatment that is to be washed off, or an out-bath type treatment that is not to be washed off).

A method of applying the hair cosmetic product of the present invention to hair is not particularly limited. It may be either a washing off method (in the case of a shampoo, a conditioner, etc.), or a leave-on method (in the case of gel, mousse, cream, lotion, spray, air-jet type styling foam, etc.).

The dose of the hair cosmetic product can be determined, as appropriate, depending on the type of an active ingredient for hair and the amount used, the amount and condition of the hair of a user, etc. In general, the present hair cosmetic product can be administered at a dose of approximately 1 µg to 50 mg/cm², and preferably approximately 2.5 µg to 10 mg/cm² , for a single administration.

The present invention will be more specifically described in the following examples. However, these examples are not intended to limit the scope of the present invention.

### Examples

### Test Example 1:

### <Preparation of casein solution 1>

1 g of casein Na (derived from milk; manufactured by Wako Pure Chemical Industries, Ltd.) was mixed into 70 mL of a 50 mM phosphate buffer (pH 10), and the pH of the obtained solution was adjusted to pH 7. The mean particle size of the aforementioned particles was measured using a light scattering photometer, Nano-ZS, manufactured by Malvern Instruments Ltd. As a result, the mean particle size was found to be 21 nm.

### <Preparation of casein solution 2>

1 g of casein Na (derived from milk; manufactured by Wako Pure Chemical Industries, Ltd.) was mixed into 70 mL of a 50 mM phosphate buffer (pH 2), and the pH of the obtained solution was adjusted to pH 3. The mean particle size of the aforementioned particles was measured using a light scattering photometer, Nano-ZS, manufactured by Malvern Instruments Ltd. As a result, the mean particle size was found to be 29 nm.

### <Preparation of casein solution 3>

1 g of casein Na (derived from milk; manufactured by Wako Pure Chemical Industries, Ltd.) was mixed into 63 mL of a 50 mM phosphate buffer (pH 2) and 7 g of 1,3-BG, and the pH of the obtained solution was adjusted to pH 3. The mean particle size of the aforementioned particles was measured using a light scattering photometer, Nano-ZS, manufactured by Malvern Instruments Ltd. As a result, the mean particle size was found to be 43 nm.

### <Preparation of casein solution 4>

1 g of casein Na (derived from milk; manufactured by Wako Pure Chemical Industries, Ltd.) was mixed into 70 mL of pure water, and the pH of the obtained solution was adjusted to pH 7. The mean particle size of the aforementioned particles was measured using a light scattering photometer, Nano-ZS, manufactured by Malvern Instruments Ltd. As a result, the mean particle size was found to be 315 nm.

### <Preparation of casein solution 5>

1 g of casein Na (derived from milk; manufactured by Wako Pure Chemical Industries, Ltd.) was mixed into 70 mL of a 50 mM citrate buffer (pH 10), and the pH of the obtained solution was adjusted to pH 7. The mean particle size of the aforementioned particles was measured using a light scattering photometer, Nano-ZS, manufactured by Malvern Instruments Ltd. As a result, the mean particle size was found to be 23 nm.

### <Preparation of casein solution 6>

1 g of casein Na (derived from milk; manufactured by Wako Pure Chemical Industries, Ltd.) was mixed into 70 mL of a 50 mM citrate buffer (pH 2), and the pH of the obtained solution was adjusted to pH 3. The mean particle size of the aforementioned particles was measured using a light scattering photometer, Nano-ZS, manufactured by Malvern Instruments Ltd. As a result, the mean particle size was found to be 27 nm.

### <Preparation of casein solution 7>

1 g of casein (derived from milk; manufactured by Wako Pure Chemical Industries, Ltd.) was mixed into 63 mL of a 50 mM citrate buffer (pH 2) and 7 g of 1,3-BG, and the pH of the obtained solution was adjusted to pH 3. The mean particle size of the aforementioned particles was measured using a light scattering photometer, Nano-ZS, manufactured by Malvern Instruments Ltd. As a result, the mean particle size was found to be 41 nm.

### <Preparation of casein solution 8>

1 g of casein Na (derived from milk; manufactured by Wako Pure Chemical Industries, Ltd.) was mixed and dispersed into 63 mL of a 1 % tartaric acid aqueous solution and 7 g of 1,3-BG, so that the pH of the obtained solution was pH 2.8. The mean particle size of the aforementioned particles was measured using a light scattering photometer, Nano-ZS, manufactured by Malvern Instruments Ltd. As a result, the mean particle size was found to be 23 nm.

### <Preparation of casein solution 9>

1 g of casein Na (derived from milk; manufactured by Wako Pure Chemical Industries, Ltd.) was mixed and dispersed into 60 mL of a 0.65% citric acid aqueous solution (pH 2.4) and 3 g of 1,3-BG, so that the pH of the obtained solution was pH 3.1. The mean particle size of the aforementioned particles was measured using a light scattering photometer, Nano-ZS, manufactured by Malvern Instruments Ltd. As a result, the mean particle size was found to be 22 nm.

### <Preparation of casein solution 10>

1 g of casein Na (derived from milk; manufactured by Wako Pure Chemical Industries, Ltd.) was mixed and dispersed into 63 mL of a 50 mM maleic acid aqueous solution (pH 1.8) and 7 g of 1,3-BG, so that the pH of the obtained solution was pH 3. The mean particle size of the aforementioned particles was measured using a light scattering photometer, Nano-ZS. manufactured by Malvern Instruments Ltd. As a result, the mean particle size was found to be 25 nm.

### <Preparation of oil phase A>

| | |
|---|---|
| Cetanol | 1.5g |
| Stearyl alcohol | 3.5g |
| Glyceryl stearate | 0.5g |
| PEG-20 tristearate | 1g |
| Polyglyceride stearate | 2g |
| Dimethicone | 3g |

The above-prepared casein solution, glycerin and water were mixed to prepare a water phase. The oil phase A and the water phase were heated to 80°C. When the temperature of each phase reached 80°C, the oil phase was mixed with the water phase, and the obtained mixture was then homogenized at 2500 rpm, for 4 minutes. The cationic substances shown in Tables 1 to 3 (NIKKOL Amideamine SV (manufactured by Nikko Chemicals Co., Ltd.). Catinal DC-80 (manufactured by TOHO Chemical Industry Co., Ltd.), Catinal BHC-60BE (manufactured by TOHO Chemical Industry Co., Ltd.), SILSTLE104 (manufactured by Dow Coming Toray Co., Ltd.), and SH556 (manufactured by Dow Coming Toray Co., Ltd.)) had previously been mixed with the oil phase A, or these substances were added after completion of the emulsification step of the oil phase A, as described in Tables 1 to 5. The coloring agents shown in Tables 3 and 5 had been added to the water phase before subjecting it to homogenization. The amount of water added was adjusted, so as to obtain a yield of 100g.The appearances of the above-prepared emulsions were each evaluated as being "emulsified" or "separated." The results are shown in Tables 1 to 5.

[Table 1]

**Table 1**

| | Comparative Example 1 | Comparative Example 2 | Example 1 | Example 2 | Comparative Example 3 | Comparative Example 4 |
|---|---|---|---|---|---|---|
| Casein solution | No. 1 70 ml | No. 1 70ml | No. 2 70 ml | No. 3 70 ml | No. 4 70 ml | No. 5 70 ml |
| Glycerin | 5 g | 5 g | 5 g | 5 g | 5 g | 5 g |
| Water | Upto 100g | Upto 100g | Upto 100g | Upto 100g | Upto 100g | Upto 100g |
| Oil phase A | 11.5 g | 11.5 g | 11.5 g | 11.5 g | 11.5 g | 11.5 g |
| NIKKOL Amideamine SV (Nikko Chemicals) | - | 2 g | 2 g | 2 g (Premixing with oil phase A) | 2g | 2g |
| Emulsification Step | Homogenizer | Homogenizer | Homogenizer | Homogenizer | Homogenizer | Homogenizer |
| pH of finished product | 6.1 | 5.9 | 6.5 | 5.5 | 6.5 | 4.6 |
| Appearance | Emulsified | Separated | Emulsified | Emulsified | Separated | Separated |

[Table 2]

**Table 2**

| | Comparative Example 5 | Example 3 | Example 4 | Example 5 |
|---|---|---|---|---|
| Casein solution | No. 5 70 ml | No. 6 70 ml | No. 7 70 ml | No. 3 70 ml |
| Glycerin | 5 g | 5g | 5g | 5 g |
| Water | Upto 100g | Upto 100g | Upto 100g | Upto 100g |
| Oil phase A | 11.5 g | 11.5 g | 11.5 g | 11.5 g |
| NIKKOL Amideamine SV (Nikko Chemicals) | 2 g | 2 g (Premixing with oil phase A) | 2 g (Premixing with oil phase A) | - |
| Catinal DC-80 (TOHO Chemical Industry) | - | - | - | 2.5 g |
| Emulsification step | Homogenizer | Homogenizer | Homogenizer | Homogenizer |
| pH of finished product | 6.5 | 6.3 | 4.2 | 7.4 |
| Appearance | Separated | Emulsified | Emulsified | Emulsified |

[Table 3]

**Table 3**

| | Example 6 | Example 7 | Example 8 |
|---|---|---|---|
| Casein solution | No. 3 | No. 3 | No. 3 |
| | 70 ml | 70 | ml 35 ml |
| Glycerin | 5 g | 5 g | 5 g |
| Water | Upto 100g | Upto 100g | Upto 100g |
| Oil phase A | 11.5 g | 11.5 g | 11.5 g |
| Catinal DC-80 (TOHO Chemical Industry) | 2.5 g (Premixing with oil phase A) | 2.5 g (Premixing with oil phase A) | 2.5 g (Premixing with oil phase A) |
| Catinal BHC-60BE (TOHO Chemical Industry) | 3.7 g (Premixing with oil phase A) | 3.7 g (Premixing with oil phase A) | 3.7 g (Premixing with oil phase A) |
| SILSTLE104 (DOW CORNING TORAY) | 1 g (Premixing with oil phase A) | 1 g (Premixing with oil phase A) | 1 g (Premixing with oil phase A) |
| SH556 (DOW CORNING TORAY) | 1 g (Premixing with oil phase A) | 1 g (Premixing with oil phase A) | 1 g (Premixing with oil phase A) |
| Black No. 401 | - | - | 150 mg |
| Purple No. 401 | - | - | 20 mg |
| Basic blue No. 99 | 300 mg | 100 mg | - |
| HC blue No. 2 | - | 150 mg | - |
| Basic brown No. 16 | 100 mg | - | - |
| HC yellow No. 4 | - | 20 mg | - |
| 4-Hydroxypropylamino-3-nitrophenol | - | 4 mg | |
| Emulsification step | Homogenizer | Homogenizer | Homogenizer |
| pH of finished product | 6.4 | 8.1 | 3.4 |
| Appearance | Emulsified | Emulsified | Emulsified |

[Table 4]

**Table4**

| | Example 9 | Example 10 |
|---|---|---|
| Casein solution | No. 8 | No. 10 |
| | 7 ml | 14 ml |
| Glycerin | 5 g | 5 g |
| Water | Upto 100g | Upto 100g |
| Oil phase A | 11.5 g | 11.5 g |
| Catinal DC-80 (TOHO Chemical Industry) | 2.5 g (Premixing with oil phase A) | 2.5 g (Premixing with oil phase A) |
| Catinal BHC-60BE (TOHO Chemical Industry) | 3.7 g (Premixing with oil phase A) | 3.7 g (Premixing with oil phase A) |
| SILSTLE104 (DOW CORNING TORAY) | 1 g (Premixing with oil phase A) | 1 g (Premixing with oil phase A) |
| Emulsification step | Homogenizer | Homogenizer |
| pH of finished product | 4.2 | 3.9 |
| Appearance | Emulsified | Emulsified |

[Table 5]

**Table 5**

| | Example 11 | Reference Example 1 | Comparative Example 6 |
|---|---|---|---|
| Casein solution | No. 9 70 ml | No. 1 1 0.35 ml | No. 1 14 ml |
| Glycerin | 5 g | 5 g | 5 g |
| Water | Upto 100g | Upto 100g | Upto 100g |
| Oil phase A | 11.5 g | 11.5 g | 11.5 g |
| Catinal DC-80 (TOHO Chemical Industry) | 2.5 g (Premixing with oil phase A) | 2.5 g (Premixing with oil phase A) | 2.5 g (Premixing with oil phase A) |
| Catinal BHC-60BE (TOHO Chemical Industry) | 3.7 g (Premixing with oil phase A) | 3.7 g (Premixing with oil phase A) | 3.7 g (Premixing with oil phase A) |
| SILSTLE104 (DOW CORNING TORAY) | 1 g with oil phase A) | 1 g with oil phase A) | 1 g (Premixing with oil phase A) |
| SH556 (DOW CORNING TORAY) | 1 g (Premixing with oil phase A) | 1 g (Premixing with oil phase A) | I g (Premixing with oil phase A) |
| Collagen (Nitta Gelatin) | 10 mg | - | |
| Astaxanthin (ASTOTS-S, Takeda Shiki) | 10 mg | - | - |
| Tocopherol (ICHIMARU PHARCOS) | 100 mg | - | - |
| Basic Blue No.99 | - | - | 300 mg |
| Basic brovvn No. 16 | - | - | 100 mg |
| Emulsification step | Homogenizer | Homogenizer | Homogenizer |
| After adjustment of pH | 3.2 | 3.5 | 6.5 |
| Appearance (Immediately after emulsification) | Emulsified | Emulsified | Emulsified |
| Appearance (After leaving at 40°C for 1 week) | Emulsified | Emulsified | Syneresis solution (separated) |

From the results shown in Tables 1 to 5, it was demonstrated that if a cationic surfactant is added under conditions in which it has a pH value greater than the isoelectric point of the casein nanoparticles (anionic particles), the mixture of the water phase and the oil phase cannot be emulsified, but that if a cationic surfactant is added under conditions in which it has a pH value less than the isoelectric point of the casein nanoparticles (cationic particles), the mixture can be emulsified.

### Test Example 2:

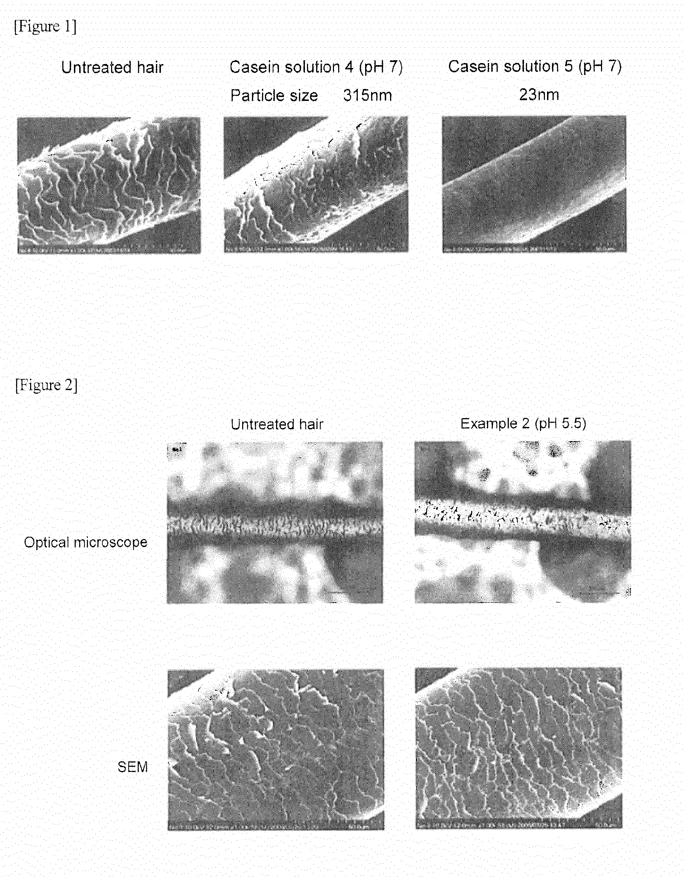

2 g of casein solution 4 or casein solution 5 was applied to I g of damaged hair (human hair), and was allowed to penetrate into the hair for 5 minutes. Thereafter, each casein solution was washed off with running water, and the damaged hair (human hair) was then observed under an electron microscope (Figure 1). The particle size was measured using "Zetasizer Nano" manufactured by Sysmex. As a result, it was confirmed that the hair treating effect of casein can be obtained by converting the casein to casein nanoparticles.

### Test Example 3:

2 g of the emulsion obtained in Example 2 was applied to 1 g of damaged hair (human hair), and was allowed to penetrate into the hair for 5 minutes. Thereafter, the emulsion was washed off with stored pure water, and the hair was then dried. On the damaged hair, Pt was deposited by evaporation, and the hair was then observed under an optical microscope and a scanning electron microscope (SEM) (Figure 2). As a result, it was confirmed that casein has ability to repair hair cuticles.

As described above, it could be confirmed that protein nanoparticles contained in the hair treatment composition produced by the production method of the present invention have ability to repair hair cuticles even in an emulsification system. In addition, it could also be confirmed that the hair treatment composition produced by the production method of the present invention, which has been applied to hair and has been then washed off, can maintain its hair treating affect.

## Claims

1. A method for producing a hair cosmetic product containing protein nanoparticles which is obtained by mixing an aqueous medium and an oil medium, wherein
(a) protein nanoparticles having a mean particle size of 10 to 200 nm are mixed with an aqueous medium having a pH value different from the isoelectric point of the protein at an amount of 0.01% by weight to 5% by weight based on the total weight of the entire solution; and
(b) the obtained liquid is mixed with a substance selected from a cationic substance and an anionic substance, which has an electric charge that is on the same side as that of the protein obtained in the step (a) .

2. The method according to claim 1, wherein, in the step (a), the protein nanoparticles having a mean particle size of 10 to 200 nm are mixed with an aqueous medium having a pH value less than the isoelectric point of the protein at an amount of 0. 0 1 % by weight to 5% by weight based on the total weight of the entire solution, and in the step (b), the obtained liquid is mixed with a cationic substance.

3. The method according to claim 1, wherein, in the step (a), the protein nanoparticles having a mean particle size of 10 to 200 nm are mixed with an aqueous medium having a pH value greater than the isoelectric point of the protein at an amount 0,01% by weight to 5% by weight based on the total weight of the entire solution, and in the step (b), the obtained liquid is mixed with an anionic substance.

4. The method according to any one of claims 1 to 3, wherein the hair cosmetic product is a hair wash, a treatment, a conditioner, or a rinse.

5. The method according to any one of claims I to 4, wherein, in the step (b), the liquid obtained in the step (a) is mixed with an oil medium and a cationic or anionic substance, so as to prepare an emulsion.

6. The method according to any one of claims 1 to 5, wherein, in the step (a), the protein nanoparticles having a mean particle size of 10 to 200 nm are mixed with either an aqueous medium having a pH value less than the isoelectric point of the protein by pH 0.5 or more, or an aqueous medium having a pH value greater than the isoelectric point of the protein by pH 0.5 or more, at an amount of 0.01% by weight to 5% by weight based on the total weight of the entire solution, so as to prepare an emulsion.

7. The method according to any one of claims 1 to 6, wherein, in the step (a), the protein nanoparticles having a mean particle size of 10 to 200 nm are mixed with an aqueous medium having a pH value different from the isoelectric point of the protein at an amount of 0.1% by weight to 5% by weight based on the total weight of the entire solution, so as to prepare an emulsion.

8. The method according to any one of claims 1 to 7, wherein the protein is at least one type selected from the group consisting of collagen, gelatin, acid-treated gelatin, albumin, ovalbumin, casein, a casein derivative, transferrin, globulin, fibroin, fibrin, laminin, fibronectin, and vitronectin.

9. The method according to any one of claims 1 to 8, which further comprises a step of mixing a silicon compound into the hair cosmetic product.

10. The method according to any one of claims 1 to 9, which further comprises a step of mixing a coloring agent into the hair cosmetic product.
